# EUROPEAN PATENT SPECIFICATION

(11) **EP 1 485 304 B1**
(45) Date of publication and mention of the grant of the patent: **09.08.2006**
(21) Application number: 03704780.0
(22) Date of filing: 11.02.2003
(51) Int. Cl.: B65D 55/02, A61B 10/00, B01L 3/00, B65D 77/04, B65D 79/02, B65D 81/26, B65D 55/06, G09F 3/00

(54) **PACKAGING**
VERPACKUNG
EMBALLAGE

(30) Priority: 19.03.2002 GB 0206456; 11.04.2002 US 120155
(43) Date of publication of application: 15.12.2004
(73) Proprietor: Royal Mail Group plc., London EC1V 9HQ (GB)
(72) Inventor: WILLIAMSON, Alistair, John, Manchester M21 9DZ (GB)
(74) Representative: Musker, David Charles
(86) International application number: PCT/GB2003/000596
(87) International publication number: WO 2003/080464

(56) References cited:
- EP-A- 0 344 966
- EP-A- 0 509 896
- WO-A-94/04425
- CH-A- 313 275
- DE-A- 3 417 371
- DE-A- 4 236 393
- DE-A- 19 603 801
- FR-A- 934 368
- FR-A- 2 745 276
- GB-A- 2 118 145
- GB-A- 2 298 413
- US-A- 3 204 849
- US-A- 3 833 113
- US-A- 5 829 594
- US-A- 6 085 927

## Description

### FIELD OF THE INVENTION

This invention relates to packaging. One particularly preferred embodiment of the invention relates to packaging for use in the transport of materials through the mail. In one illustrative embodiment these materials are hazardous materials such as blood or other bodily fluids.

The present invention, and its advantages over the prior art, will now be described with particular reference to the transport of hazardous materials, such as blood samples or the like, through the mail. However, it will be apparent to persons skilled in the art that the teachings of the invention will prove useful in a variety of different circumstances. As a consequence, the following description should not be read as limiting the scope of the invention in any way.

### BACKGROUND TO THE INVEN1ZON

As an alternative to hand delivery or expensive courier services, it has previously been proposed to transport materials, such as blood samples for example, by mail from a location where the samples are obtained (such as a Doctor's surgery or hospital for example) to another location (such as a laboratory) where tests can be undertaken upon the samples supplied.

For these tests to be undertaken, the packaging employed in the transport of the samples must be sufficiently robust to ensure that the samples are in a good condition when the laboratory receives them. It is also desirable, in the event that the samples have been damaged during transit, for that damage to be apparent when the packaging is first opened so that the likelihood of the individual opening the package coming into contact with the materials, or injuring themselves on the damaged packaging, is reduced.

It is also preferred for the packaging to be shaped so that it can be posted through a conventional letterbox in order to avoid delaying delivery of the package simply because the recipient's premises are not open when delivery is attempted. This is of particular importance given the recently proposed reduction in the number of daytime postal deliveries in the United Kingdom. An appropriately shaped package would also be convenient for the sender since such a package would not have to be taken to a post office for posting, but could instead be posted through the posting slot of any conventional mailbox.

Another preferred requirement for packaging is that it must be difficult, and preferably impossible, for third parties to interfere with the samples within the packaging without detection of the interference by the recipient. For example, if blood samples have been taken as part of a drug testing programme for athletes, then no-one but the appropriate authorities should be able to gain undetected access to the samples if the results of the testing are to be legally enforceable.

United Kingdom Patent Application No. 2,184,421 discloses a mailer for blood products that is typical of previously proposed packaging. The mailer described in this document comprises an insulating foam bag in which glass ampoules containing blood are held by a series of foam tubes that have been joined to one another. The tubes are held in place within the bag by wedge-shaped packing bags, and the mailer is closed by two safety closures with backwardly facing barbs that resist accidental removal.

A problem with this mailer is that whilst it does provide some protection to the ampoules, it is still possible to break those ampoules by flexing or crushing the packaging. It is also possible to remove the safety closures and then reapply them without detection. Another problem with this mailer is that it is not possible to determine whether the ampoules are intact before the mailer is opened, and as a consequence there is a significant risk of injury to the person opening the mailer.

United Kingdom Patent Application No. 2,213,457 discloses packaging which is more robust than that proposed in UK Application No. 2,184,421, but which still suffers from problems associated with verifying that the contents are intact before opening, and that they have not been interfered with during transit.

United States Patent Application No. 5,620,097 discloses a mailer that is designed to facilitate the transport of thin, generally flat substrates bearing dried blood spots, for example, through the mail. The mailer disclosed in this document, if adapted for the transport of larger samples, would also be affected by some of the problems mentioned above, in particular those affecting the packaging described in UK Patent Application No. 2,213,457.

It is an aim of the present invention to avoid, or at least reduce, some or all of the problems described above.

### STATEMENT OF INVENTION

In pursuit of this aim, one embodiment of the invention provides packaging for use in the transport of an item through the mail, the packaging comprising first and second components that can be assembled to define an enclosed storage cavity for the item to be transported, wherein said components are each provided with respective complementary parts of an engagement mechanism, said packaging further comprising an opener operable to weaken a peripheral wall of a said component, to enable said component to be broken open, wherein said components cannot be reassembled once said opener has been operated, the packaging characterised by a structure operable to obstruct access to the engagement mechanism from outside the packaging.

By virtue of this arrangement it is easy for a recipient of the packaging to determine whether or not an attempt has been made to tamper with the contents during transit.

Preferred features of these embodiments are set out in the dependent claims. Other advantages of the embodiments are set out elsewhere in the description.

### BRIEF DESCRIPTION OF THE DRAWINGS

Embodiments of the invention will now be described, by way of example only, with reference to the accompanying drawings, in which:
*Fig. 1* is a schematic perspective view of the front of packaging in accordance with an embodiment of the invention;
*Fig.* 2 is a schematic perspective view of the rear of the packaging shown in Fig. 1;
*Fig. 3* is an exploded top perspective view of the inside of the packaging shown in Figs 1 and 2;
*Fig. 3a* is an enlarged partial section along the line a―a in Fig. 1;
*Fig. 4* is an exploded top perspective view of the packaging of Fig 3 with an anti-lock insert removed;
*Fig. 5* is an exploded bottom perspective view of the packaging;
*Fig. 6* is a perspective view of the inside of a top part of the packaging;
*Figs. 7a to 7g* are various views of a bottom part of the packaging;
*Figs. 8a to 8f* are various views of an inner receptacle of the packaging;
*Fig. 9a* is a schematic representation of a sample bottle;
*Fig 9b* is a schematic illustration of sample bottle that has been wrapped in an absorbent covering and placed inside a clear press-sealable bag;
*Figs. 10a to 10d* are schematic views of the assembled packaging (including sample bottles) before posting;
*Fig. 11* is a plan view of the underside of an adhesive label fixable to the outside of the packaging;
*Fig. 12* is a perspective view of the packing with the label of Fig. 11 partly affixed thereto;
*Figs. 13 and 14* are front and rear views, respectively, of the packaging after all of the label shown in Figs. 11 and 12 has been adhered to the packaging; and
*Fig. 15* is a plan view of an illustrative alternative construction for the inner receptacle shown in Figs 8a to 8f.

### DETAILED DESCRIPTION OF PREFERRED EMBODIMENTS

Embodiments of the invention illustrating presently preferred best modes for practising the invention will now be described with particular reference to the use of the packaging as a mailer for hazardous materials such as blood samples. It will be appreciated that the mailer can be used for a variety of different functions, and in a variety of different ways and thus that the following description should not be read as limiting the scope of the invention in any way.

As will now be explained in detail, the packaging of the preferred embodiment comprises an inner receptacle that is secured within the bottom part of a two-part outer housing. The packaging is supplied with an insert that must be removed before the packaging can be used. The insert is provided to prevent respective complementary components of an engagement mechanism from engaging to lock the top part to the bottom part until the packaging is ready to be placed in the mail. Preferably the outer housing and inner receptacle are formed by injection moulding, and are of polypropylene and clear polycarbonate respectively. In a highly preferred embodiment, the packaging of the invention is of a material that can be recycled.

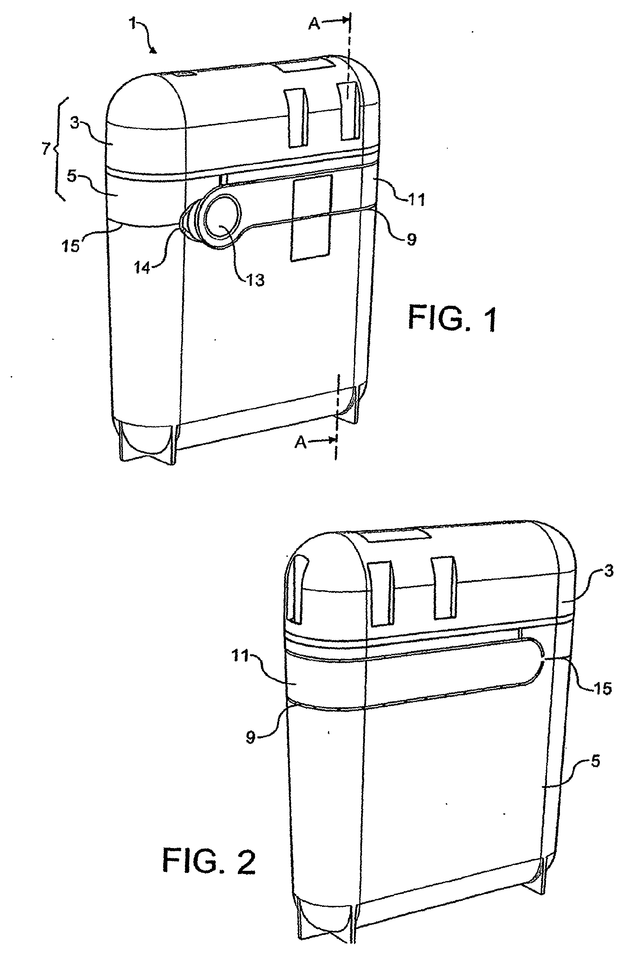

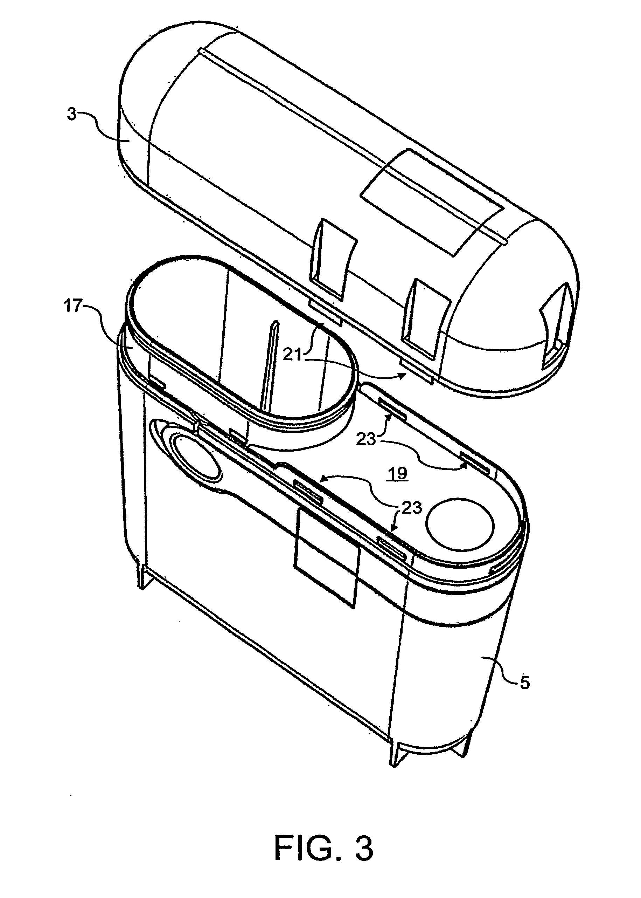

As mentioned above, Fig. 1 is a schematic perspective view of the front of an item of packaging 1 in accordance with an embodiment of the invention. The packaging illustrated in Fig. 1 is in an unlocked state where a top part 3 and a bottom part 5, that together define an outer housing 7, can be freely separated. Fig. 2 illustrates the rear of the packaging shown in Fig. 1.

As shown in the drawings, the packaging is generally rectangular in cross section so that it can easily be fitted through most normal posting box apertures. As an example, for United Kingdom posting boxes it is preferred that the packaging is no thicker than 50mm, no wider than 150mm and no taller than 180mm. These dimensions may be varied at will to account for differently shaped posting slots in different countries.

Clearly visible in Figs. 1 and 2 is a continuous line 9 that comprises a line of weakness in the outer housing 7. As shown in Figs 1 and 2, the line 9 defines a generally rectangular strip 11 that extends from the front of the packaging, and around one side of the packaging to the rear thereof. One end of the strip 11 (otherwise referred to herein as an opener) is formed with a pull-tab 13 adjacent a half-moon shaped aperture 14 in the outer housing that is provided to make it easier for a user to grasp the pull tab when the packaging is opened (as will later be described). Joining either end of the strip 11, and extending around the other side of the packaging is another line of weakness 15 that enables the top part 3 to be separated from the bottom part 5 when the packaging is opened.

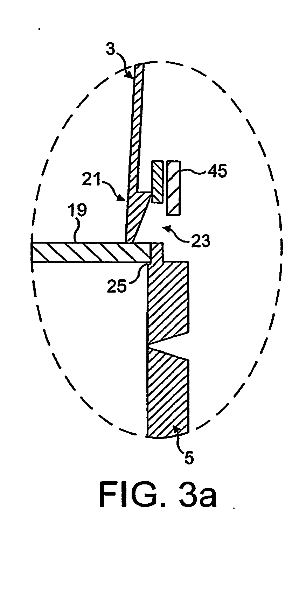

Fig. 3 is an exploded top perspective view of the inside of the packaging 1 shown in Figs 1 and 2. As shown, the packaging includes an inner receptacle 17 that is secured (as will later be described) within the bottom part 5 of the outer housing 7. Clearly visible in Fig. 3 is an anti-lock insert 19 that functions to prevent respective complementary components of an engagement mechanism from engaging to lock the top part 3 to the bottom part 5 until the packaging is ready to be used. Just visible below the bottom edge of the top part 3 are end portions of two of a number of barbed resilient tabs 21 which form the component of the aforementioned engagement mechanism provided on the top part 3 of the packaging 1. As will later be described, the barbed tabs 21 can engage with corresponding slots 23 (forming the component of the aforementioned engagement mechanism provided on the top part 3 of the packaging 1) to lock the top part 3 to the bottom part 5.

Fig. 3a is an enlarged partial section along the line a―a in Fig. 1 that illustrates how the insert 19 (which could be of cardboard or plastic for example) functions to prevent the top part 3 from locking to the bottom part 5. The insert 19 is shaped to rest on an internal lip 25 formed about the inside of the peripheral edge of the bottom part 5. With the insert placed within the bottom part 5 the barbs on the resilient tabs 21 are prevented from engaging with the slots 23, and as a consequence the top part 3 cannot be locked to the bottom part 5.

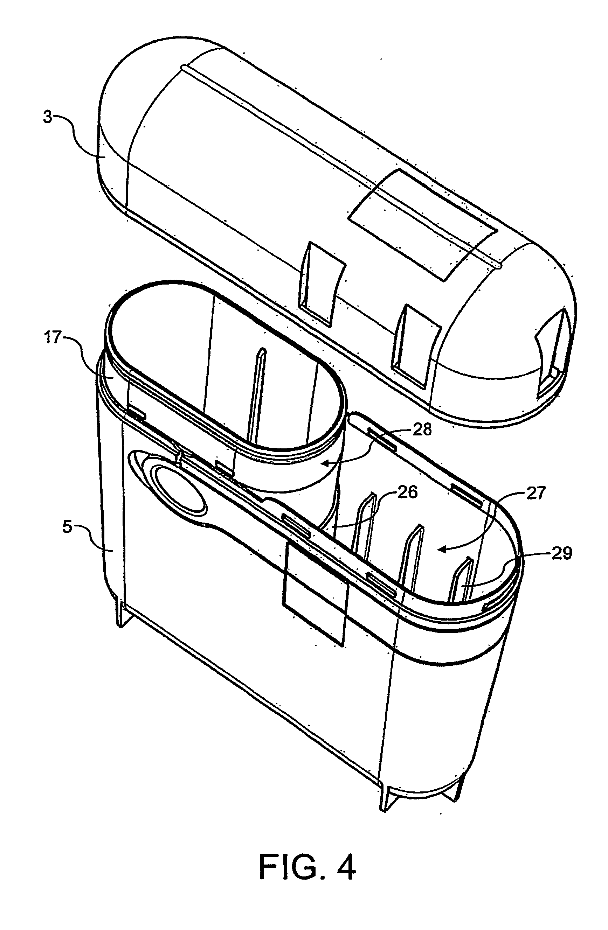

Fig. 4 is an exploded top perspective view of the packaging of Fig 3 with the anti-lock insert 19 removed. The inside of the bottom part 5 is divided in two by a curved medial wall 26 to define a first cavity 28 in which the internal receptacle 17 can be fitted, and a second cavity 27 that is provided with a number of structural supports 29 which help to strengthen the packaging against damage.

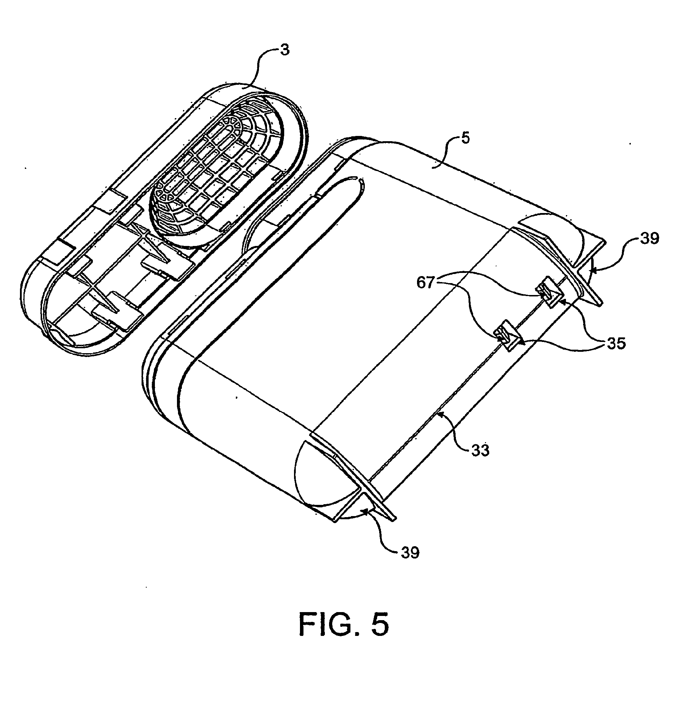

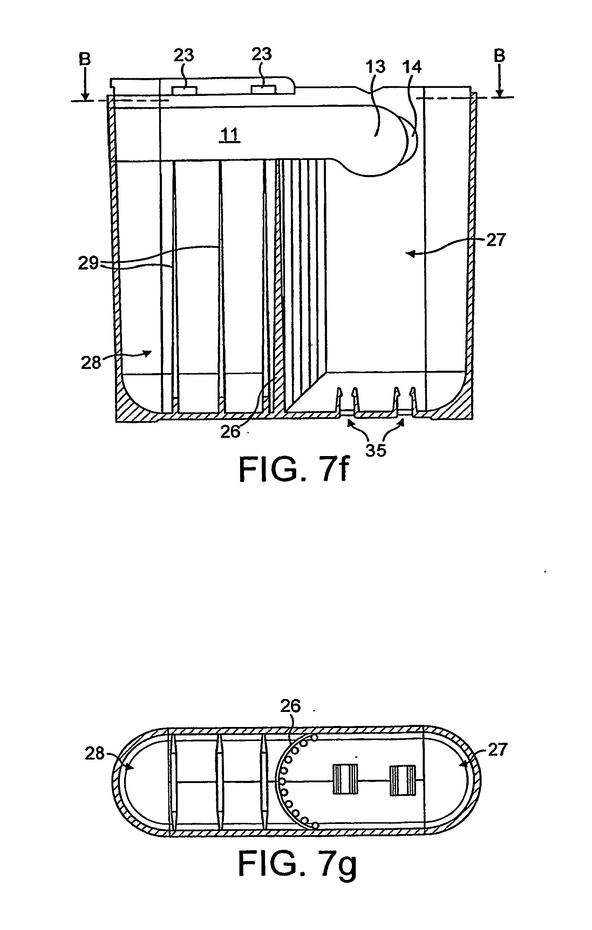

Fig. 5 is an exploded bottom perspective view of the packaging 1. As shown, the underside 33 of the packaging 1 has two openings 35 with barbed internal walls (see Fig. 7f for example) that engage with barbed tabs 67 depending from a bottom wall of the inner receptacle, to hold the inner receptacle within the packaging 1. Each end of the underside of the packaging is formed with a generally T-shaped structure 39 that allows the bottom part 5 of the packaging 1 to be stood upright.

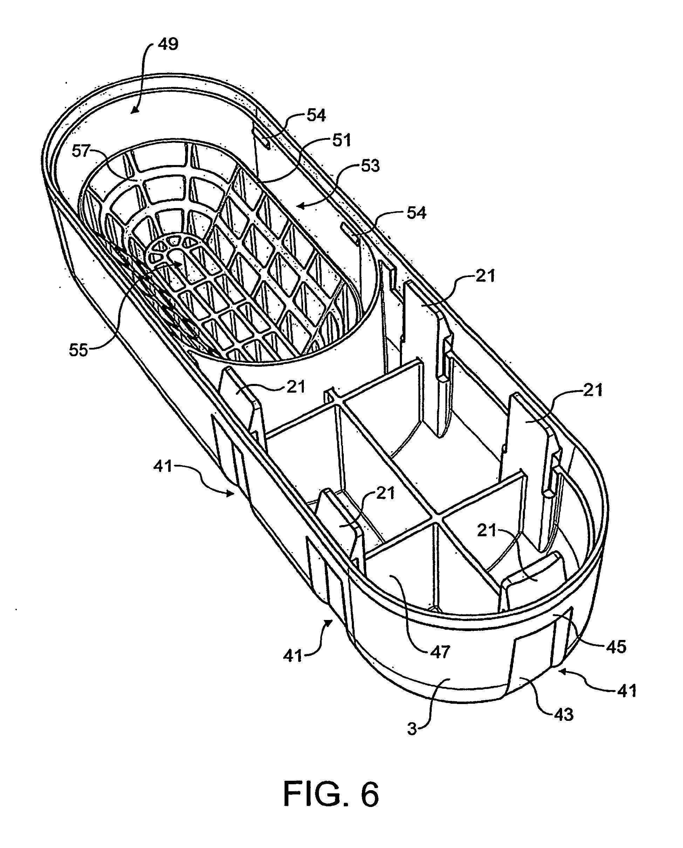

Fig. 6 is a perspective view of the inside of the top part 3 of the packaging 1. A number of indented channels 41 are formed in the peripheral wall of the top part, and the above described barbed tabs 21 each extend from a back wall 43 of a respective indented channel behind a portion 45 of the top part peripheral wall that bridges each of the aforementioned channels 41. The bridging portions 45 function to obstruct access to the barbs 21 from outside of the packaging when the barbs are secured in the slots 23. The barbed tabs are interconnected by a series of structural reinforcing walls 47 that are provided to increase the strength of the top part.

A cap 49 is formed inside one end of the top part, and a peripheral wall 51 of the cap 49 is spaced from the peripheral wall of the top part to form a slot 53 that the open end of the inner receptacle 17 can fit into when the top part 3 and the bottom part 5 are fitted together. The cap 49 has a domed internal cavity 55 formed by a number of interconnected reinforcing walls 57 to increase the strength of the cap. A number of detents 54 (two of which are visible) are formed on the inside of the peripheral wall of the top part 3, and extend inwardly from the peripheral wall into the slot 53.

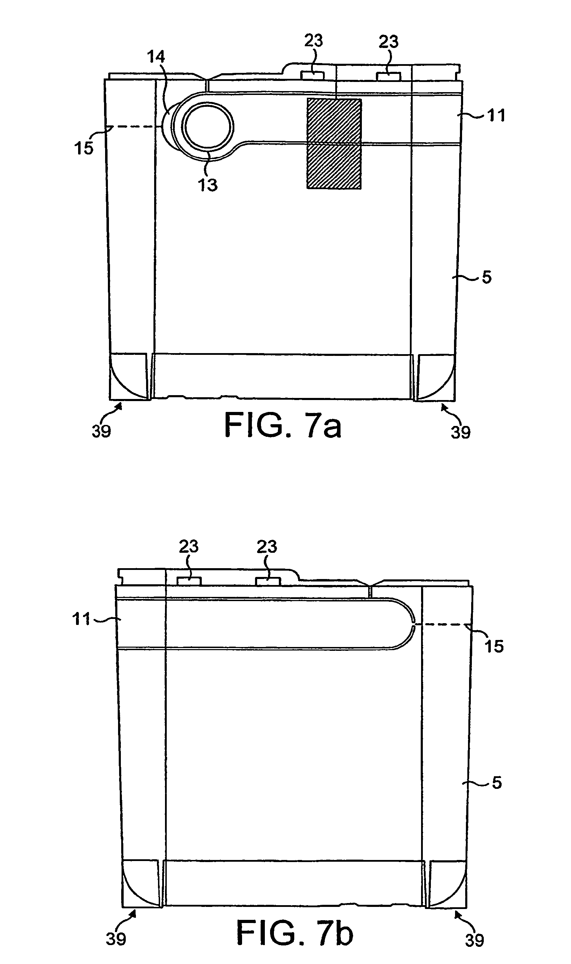

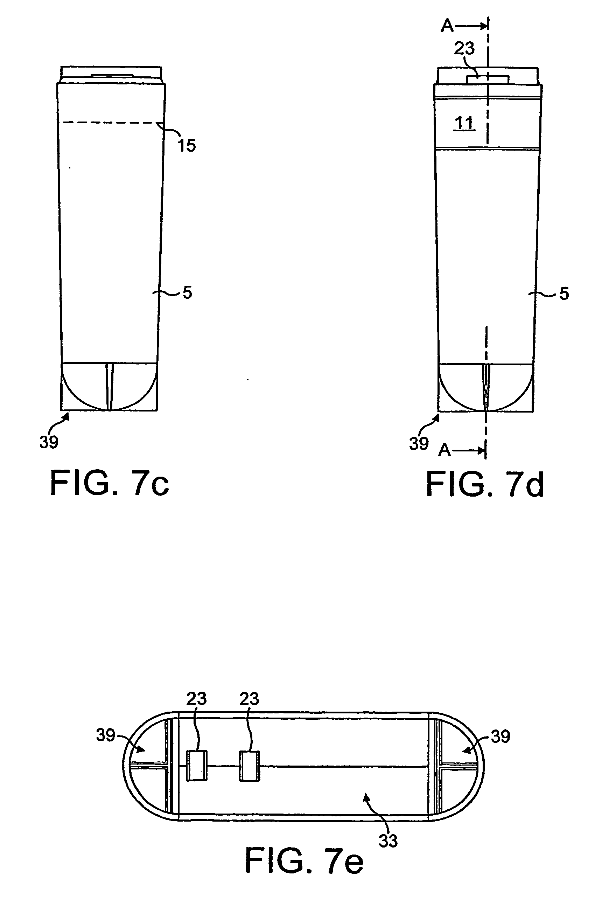

Figs 7a, 7b, 7c and 7d are, respectively, schematic elevations of the front of the bottom part 5, the back of the bottom part 5, the left side of the bottom part 5, and the right side of the bottom part 5. Fig. 7e is an underneath plan view of the bottom part 5 showing the T-shaped structures 39 and slots 35.

Fig. 7f is a cross-sectional view along the line A―A in Fig. 7d showing the curved medial wall 25 that defines the first cavity 26 in which the internal receptacle 17 can be fitted, and the second cavity 27. Also visible are the reinforcing structural walls 29, the aperture 14 in the outer housing formed alongside the pull tab 13, and the barbed internal walls 59 of the two openings 35 in the underside 33 of the bottom part 5. Figure 7g is a top plan view of the inside of the bottom part along the line B―B in Fig. 7f.

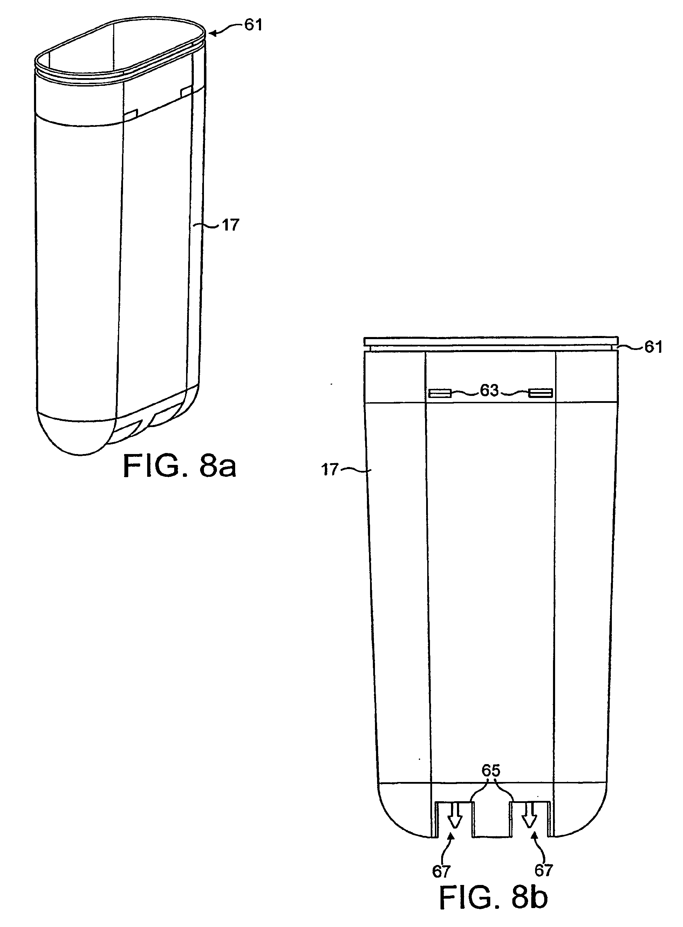

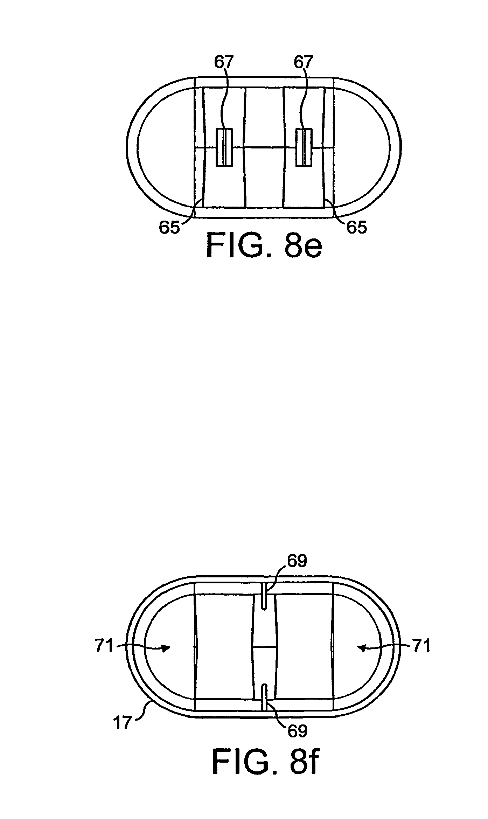

Fig. 8a is a perspective view of the inner receptacle 17. As shown, the receptacle is hollow, and generally cylindrical with one open end and another closed end.

Fig. 8b is an elevation of the inner receptacle 17. In the preferred embodiment the receptacle is formed of clear polycarbonate so that the contents of the receptacle can be viewed before they are removed. The receptacle is also formed with a channel 61 running around the outside of the peripheral wall of the receptacle close to the open end thereof. The channel 61 is sized to accept an o-ring (not shown) that forms a seal against the inside of the slot 53 when the top and bottom parts of the packaging are fitted together. A number of notches 63 are formed in the peripheral wall of the receptacle, and are positioned so that they engage with the detents 54 projecting inwardly from the peripheral wall of the top part 3 when the top part is coupled to the bottom part.

The closed end of the receptacle is formed with a pair of recesses 65, and a barbed projection 67 is formed in each recess. The barbed recesses are arranged to engage with the barbed internal walls 59 of the two openings 35 in the underside 33 of the bottom part 5 to secure the inner receptacle to the bottom part 5. Fig 8e is a plan view of the underside of the closed end of the inner receptacle.

In the preferred arrangement the strength of the coupling between the inner receptacle 17 and the bottom part 5 is greater than the strength of the coupling between the detents 54 and notches 63. This arrangement is preferred so that the inner receptacle 17 will decouple from the top part 3 (when the packaging is opened) before the inner receptacle 17 will decouple from the bottom part 5. In an alternative embodiment, where is desired to extract the receptacle from the packaging before decoupling it from the top part 3, the opposite arrangement could instead be provided.

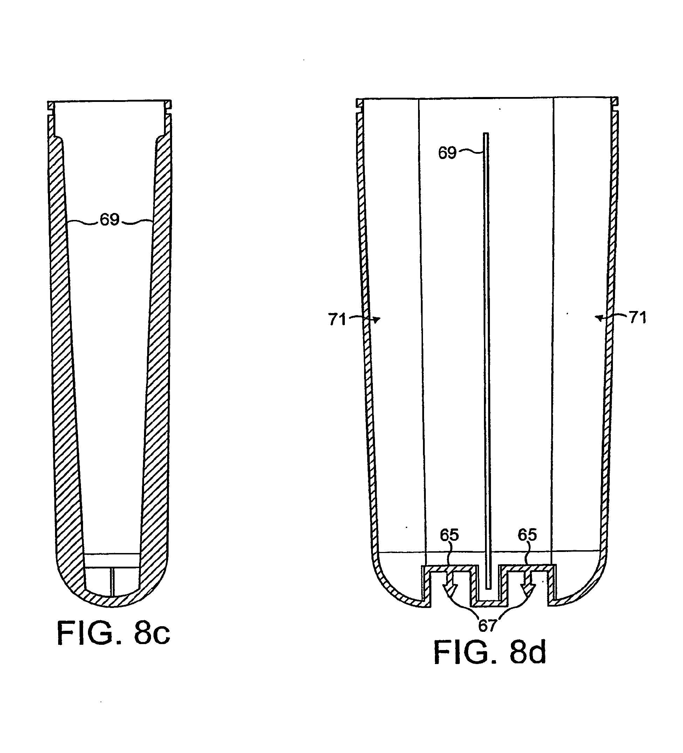

Figs. 8c and 8d are lateral and longitudinal cross-sectional views, respectively, illustrating a pair of reinforcing internal walls 69 that increase the strength of the inner receptacle (by bracing the peripheral wall of the receptacle), as well as serving to divide the interior of the inner receptacle into two compartments 71. Fig. 8f is a plan view of the inner receptacle showing the internal walls 69 and compartments 71.

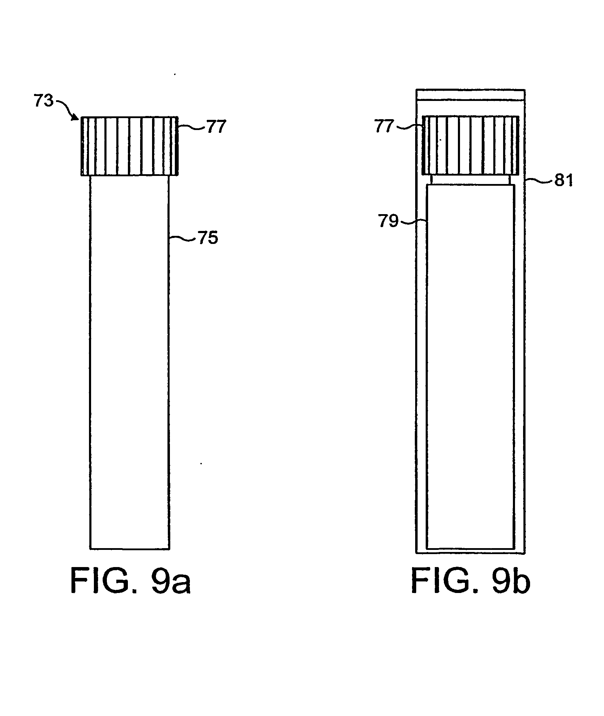

Fig. 9a is a schematic representation of a sample bottle 73 of the type commonly used for blood samples, urine samples, or other potentially hazardous samples. The bottle 73 comprises a hollow cylinder 75 that is closed by a cap 77 screw-threaded or snap-fitted thereon. As shown in Fig 9b, in the preferred embodiment the bottles are wrapped in an absorbent or super-absorbent covering 79 which is impregnated with appropriate chemicals to induce a colour change on contact with fluid of the type enclosed within the bottle. The bottle and absorbent covering are then placed inside a clear, press-sealable bag 81 (such as a plastic or polythene bag for example) that is sealed before being placed in one of the compartments 71 of the inner receptacle 17. This arrangement is preferred because the clear bag allows the individual opening the bag to immediately see whether there has been a colour change in the absorbent covering indicative of a leak from the bottle.

As an alternative to providing a covering that has been impregnated with chemicals it may simply be sufficient to colour the covering so that the contents of the bottle would be visible if they were to be spilt onto the covering. For example, if the sample were blood then the covering could be dyed white so that the red blood is clearly visible if it should leak from the bottle during transit.

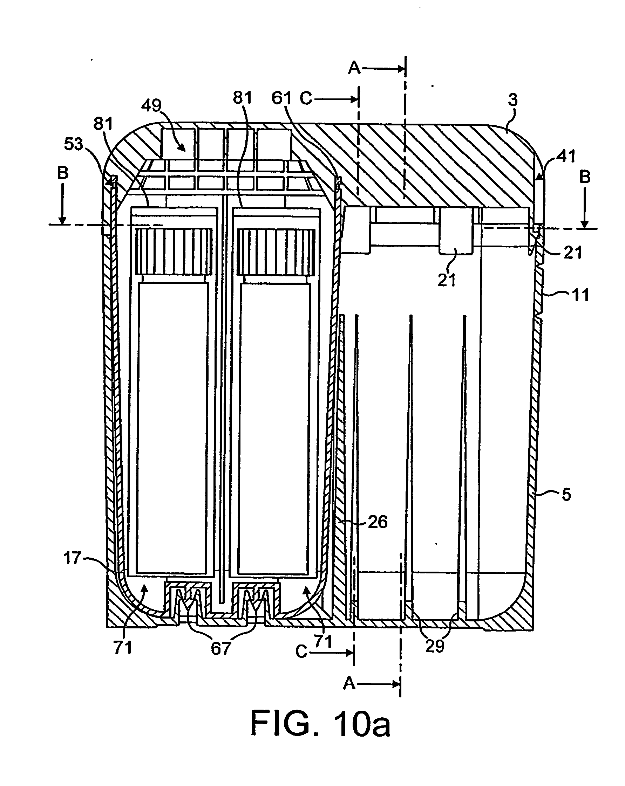

Fig 10a is a longitudinal cross sectional view of packaging in which a pair of sample bottles sealed in clear bags (as shown in Fig. 9b) have been placed in respective compartments 71 of the inner receptacle 17 before snap fitting the top part 3 to the bottom part 5. As shown, the open end of the receptacle is received in the groove 53 shown in Fig. 6, and the O-ring provided within the channel 61 formed around the outside of the receptacle serves to seal the inside of the cap 49 and receptacle 17 from the remainder of the packaging.

Fig. 10b is a cross-sectional view along the line A―A in Fig. 10a showing the extent of the second cavity 27 within the bottom part 5. The cavity 27 can be used for a variety of different purposes, and is ideal for storing any paperwork associated with the samples (such as paperwork identifying the individual providing the sample).

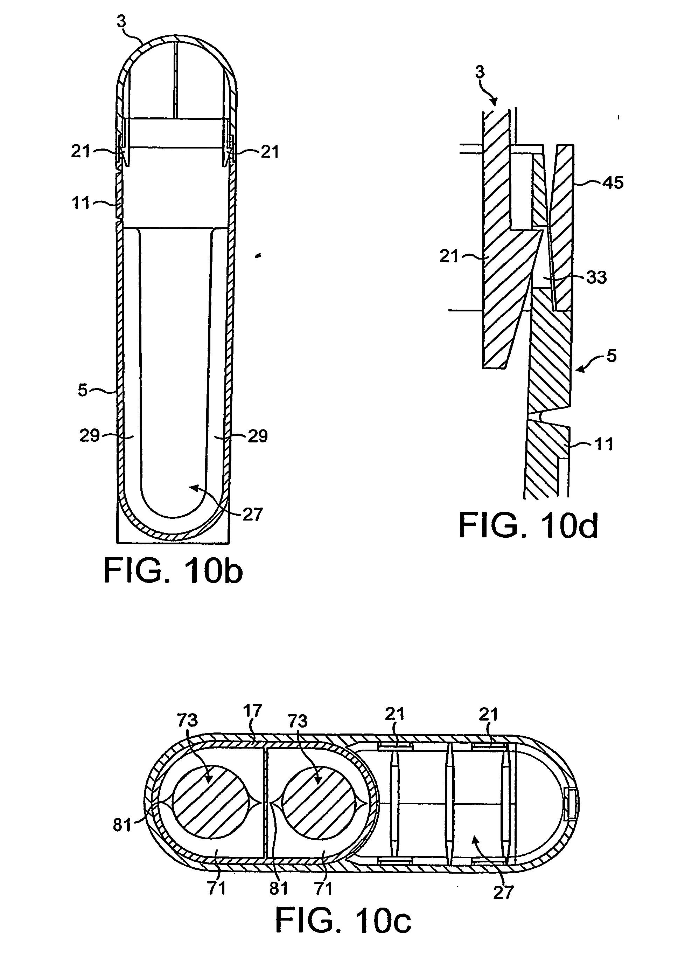

Fig. 10c is an enlarged cross-sectional view along the line B―B in Fig. 10a, and provides a plan view of the interior of the packaging 1, showing the two compartments 71 in the receptacle 17, the two press-sealable bags 81 (and sample bottles 73), and the second cavity 27.

Fig. 10d is an enlarged partial sectional view along the line C―C through a barbed tab 21 and associated slot 23 that have engaged as the top 3 and bottom 5 parts of the packaging 1 have been fitted together. As shown, the bridging portion 45 (explained in detail above in connection with Figure 6) has obscured the slot 23 so that access to the barbs 21 from outside of the packaging when the barbs are secured in the slots 23 is obstructed. In practice this means that it is very difficult, if not impossible, for third parties to disengage the barbed tabs from the slots (to open the packaging) without damaging or marking the packaging in some way.

In the preferred embodiment the arrangement is such that when the top part has been snap-fitted onto the bottom part, the sealing O-ring on the receptacle and the snap-fit tab and slot couplings provide a substantially waterproof seal which can only be broken by tearing off the generally rectangular strip 11 that extends from the front of the packaging, and around one side of the packaging to the rear thereof. As a consequence of this arrangement it is difficult, and probably impossible, for third parties to tamper with the contents of the packaging without that tampering being evident.

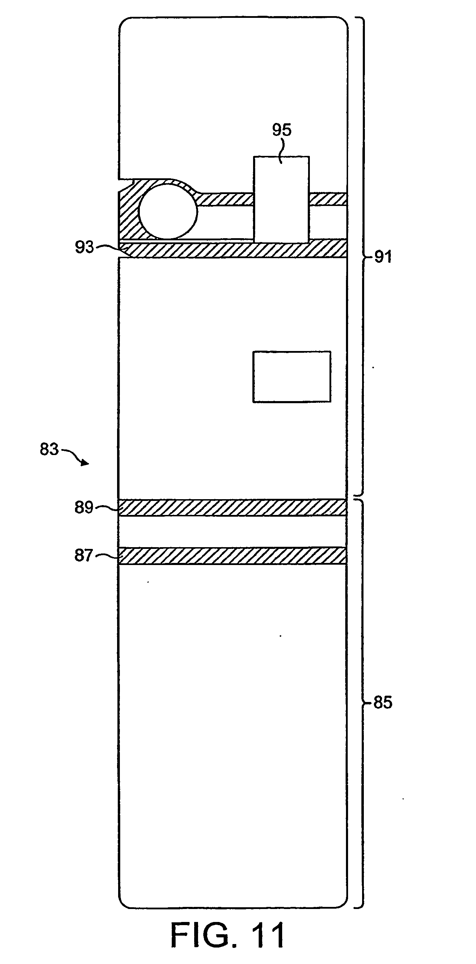

Fig. 11 is a plan view of the underside of a self-adhesive label 83 fixable to the outside of the packaging, as will now be described, to provide an additional means of preventing undetected tampering with the contents of the packaging 1. The adhesive label comprises an outer surface (of paper for example) which is coupled to one or more release papers by a layer of adhesive that can be revealed by pealing away the release paper(s). Once the adhesive has been revealed the label can be stuck to the outside of the packaging. The outer paper surface is provided with areas (not shown) for the user to insert the address of the recipient, and optionally the address of the sender.

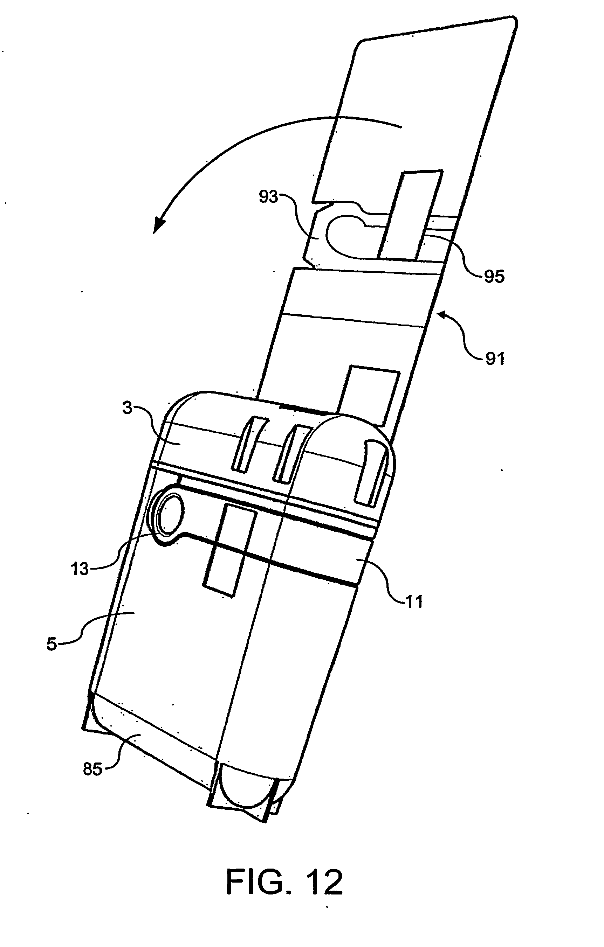

The label 83 comprises a first portion 85 which is normally affixed to the back of the bottom part of the packaging (as shown in Fig 12) by the manufacturer of the packaging before it is delivered to the customer for use. The first portion includes a pair of parallel rectangular panels 87, 89 (shown shaded in Fig 11) extending from one side of the label 83 to the other which are not backed with adhesive. Each rectangular panel is provided with a series of perforations, and is arranged to overlie the line of weakness forming the strip 11 (see Fig 2) on the back of the packaging 1. The remaining portion 91 of the label 83 is backed with release paper, which can be peeled off by a user so that they can adhere the remaining portion of the label to the front of the packaging.

As shown, the remaining portion 91 includes a generally C-shaped section 93 (shown shaded) extending from one side of the label 83 to the other. As with the rectangular panels 87, 89 mentioned above, the C-shaped section is not backed with adhesive. A line of perforations is provided, and the c-shaped section is arranged to overlie the line of weakness forming the strip 11 and pull-tab 13 (see Fig 2) when the remaining portion 91 is adhered to the front of the packaging 1.

The label 83 also comprises an aperture 95 which is provided to ensure that postage stuck onto the outside of the packaging, or pre-paid postage indications printed or otherwise formed on the outside of the packaging, are still visible once the remaining portion of the label has been stuck down.

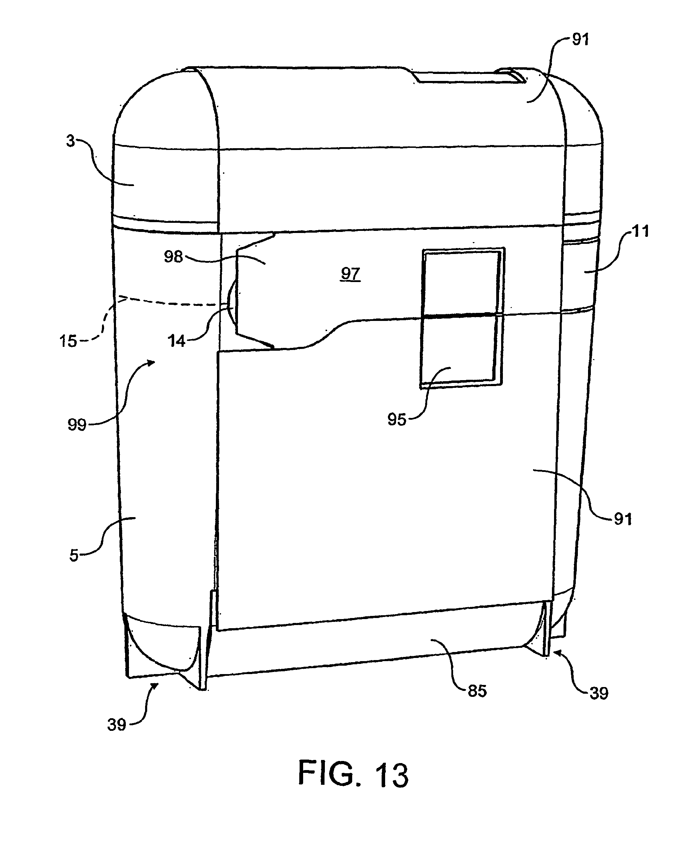

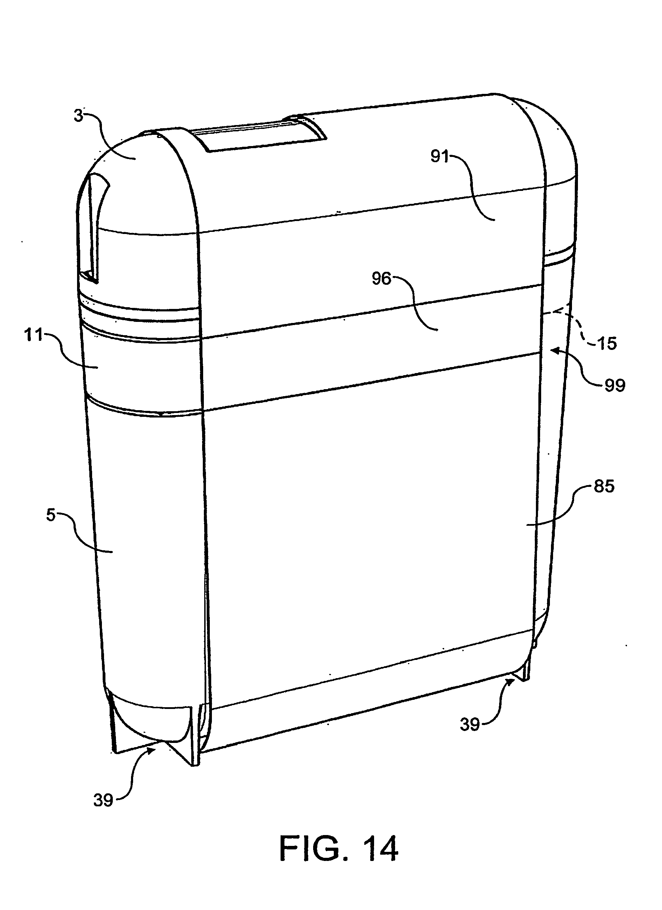

Fig. 12 is a perspective view of the packaging with the label of Fig. 11 partly affixed thereto (i.e. a view of the packaging in the form in which it most likely will be delivered to customers for use). Once the user has packed the sample bottles in the clear bags (described above in relation to Figure 9b), the bags can be placed in the receptacle and any accompanying paperwork can be placed in the neighbouring second cavity (see Fig 10a for example). The top part can then be snap-fitted onto the bottom part to permanently connect the one to the other. At this point, the user can remove the release paper on the underside of the remaining portion, and the remaining portion can be folded down (in the direction indicated by the arrow in Figure 12) and adhered to the front of the packaging. Figs. 13 and 14 are front and rear views, respectively, of the packaging after the label shown in Figs. 11 and 12 has been adhered thereto. Clearly visible are tear-off strips 96, 97 that are bordered, respectively, by the rectangular panels and the C-shaped section mentioned above (neither of which are visible). Also clearly visible is the aperture 95 through which a postage mark can be viewed.

Once the packaging has been appropriately addressed and the appropriate postage has been applied (if required), the packaging can then be consigned to the mail simply by posting it through the posting slot of any posting box.

The recipient of the packaging, on receipt thereof, can immediately see whether the packaging has been tampered with in transit by inspecting the label to see if any attempt has been made to remove it. If the label is damaged, then a second inspection may be required to see whether an attempt has been made to prise or decouple the top part away from the bottom part.

If the packaging appears not to have been tampered with, the user can then proceed to open it by lifting an end 98 of the C-shaped section (which is without adhesive - as shown in Fig 11) to reveal the half-moon shaped aperture 14. The user can then gain access to an extreme edge of the pull-tab 13 via the aperture 14, and pull the pull-tab 13 away from the front face of the packaging. The user can then continue to pull on the pull-tab 13 to peel the strip 11 and the tear-off strips 96, 97 of the label away from the packaging (by breaking the lines of weakness bounding the rectangular strip 11). When the strip has been removed the top part 3 is secured to the bottom part only by a column 99 of the packaging that incorporates the aforementioned line of weakness 15 (see Figs 1 and 2).

At this stage a preliminary check on the contents of the inner receptacle 17 can be made by looking through the space in the packaging where the strip 11 used to be, and through the clear (i.e. transparent) wall of the receptacle to see if the covering 79 visible through the bags 81 has been discoloured. If the covering is discoloured then a leak has occurred during transit and the user knows not to open the packaging, or at least to take extra care when opening the packaging.

If the covering 79 is not discoloured, then the user can be confident that a leak has not occurred, and can proceed to separate the top and bottom parts of the packaging. Separation is accomplished by pulling the remaining portion of the top part away from the remaining portion of the bottom part until the line of weakness 15 in the column 99 breaks whereupon the top part can be removed.

The user is then able to gain access to the bags containing the samples and to the accompanying paperwork (if any is supplied). Advantageously, the bottom part is free standing ― by virtue of the aforementioned T-shaped structures 39 ― and thus provides a handy storage compartment for the samples and paperwork whilst the samples are tested. This arrangement also helps to make sure that the samples do not become separated from their accompanying paperwork.

It is apparent from the above, that the packaging of the preferred embodiment provides a robust means for transporting hazardous and/or delicate samples through the mail. A chief advantage of the packaging of the preferred embodiment is that it is provided with two anti-tamper devices that provide an immediate indication to someone receiving the packaging of any attempt to tamper with the contents. An associated advantage of this is that the packaging can only be used once, and as such there is no possibility of a given item of packaging with a prepaid postage indication being used repeatedly thereby defrauding the postal services. A further advantage is that the packaging permits at least a cursory check to be made on the contents of the packaging before the contents are opened. Other advantages of the preferred embodiment will be apparent from the above description.

It will also be understood that modifications may be made to the particular embodiments described herein without departing from the spirit and scope of the invention.

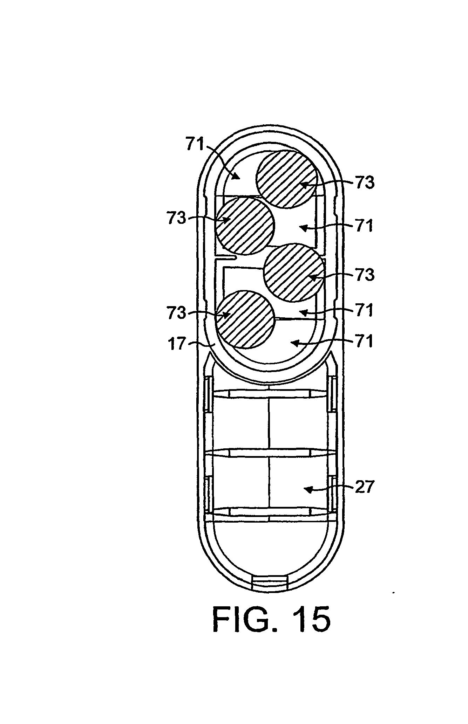

For example, Fig 15 is a plan view of one such modification where the inner receptacle has been arranged to permit four samples to be stored therein. The receptacle can be further modified, as required, to permit a fewer or greater number of samples (i.e. one or more samples) to be stored therein.

As another example, it will be apparent to persons skilled in the art that the tear strip may extend about substantially the entire periphery of the bottom part, or in other words that it is not necessary for the tear strip 11 to leave the aforementioned column 99 of material.

## Claims

1. Packaging (1) for use in the transport of an item through the mail, the packaging (1) comprising first (3) and second (5) components that can be assembled to define an enclosed storage cavity for the item to be transported, wherein said components (3, 5) are each provided with respective complementary parts of an engagement mechanism (21, 23), said packaging further comprising an opener operable to weaken a peripheral wall of a said component (5), to enable said component (5) to be broken open, wherein said components (3, 5) cannot be reassembled once said opener (11) has been operated, the packaging (1) **characterised by** a structure (45) operable to obstruct access to the engagement mechanism (21, 23) from outside the packaging.

2. Packaging according to claim 1, wherein said engagement mechanism is such that said components are locked together when assembled to define said cavity.

3. Packaging according to claim 2, wherein said engagement mechanism comprises a plurality of tabs each carrying a barb that is engageable with a corresponding slot.

4. Packaging according to claim 3, comprising a structure that is operable to prevent the barbs from being disengaged from the slots.

5. Packaging according to claim 4, wherein said structure for preventing the barbs from being disengaged from the slots comprises a plurality of peripheral wall portions of a said component inside of which said barbs extend, said peripheral wall portions being arranged to obscure the slots on the other of said components when the barbs on the tabs are engaged therewith.

6. Packaging according to claim 1, wherein one of said components is configured as a freestanding base component, said base component comprising one or more stabilisers capable of supporting the base component in an upright position.

7. Packaging according to claim 1, comprising a receptacle provided within the storage cavity, said item to be transported being storable within said receptacle.

8. Packaging according to claim 7, wherein a peripheral wall of said receptacle is, at least in part, translucent or transparent so that at least part of said item can be viewed therethrough.

9. Packaging according to claim 8, wherein all of said receptacle is translucent or transparent.

10. Packaging according to claim 7, wherein said receptacle and one of said components include respective complementary parts of an engagement mechanism that is operable to couple the receptacle to that component.

11. Packaging according to claim 10, wherein said one component is configured as a freestanding base component, said base component comprising one or more stabilisers capable of supporting the base component in an upright position, and said receptacle is capable of being coupled to said base component.

12. Packaging according to claim 11, wherein said receptacle is arranged to form a seal with a complementary formation formed as part of said second component when said components are assembled to form said storage cavity.

13. Packaging according to claim 1, wherein said item to be transported comprises a sample bottle.

14. Packaging according to claim 13, wherein said sample bottle is wrapped in an absorbent covering and received within a transparent press-sealable bag.

15. Packaging according to claim 14, wherein said absorbent covering is arranged to exhibit a colour change in the event that the contents of said sample bottle should leak therefrom and come into contact with said covering.

16. Packaging according to claim 15, wherein said covering is impregnated with a chemical that reacts with the leaked contents of said sample bottle to provide said colour change.

17. Packaging according to claim 1, wherein said opener comprises a tear strip in a peripheral wall of a said component, said tear strip being operable to remove at least a segment of the peripheral wall.

18. Packaging according to claim 17, wherein said tear strip is operable to remove a generally rectangular segment extending about the entire circumference of the component.

19. Packaging according to claim 17, wherein said tear strip is operable to remove a generally rectangular segment extending about only a portion of the circumference of the component, a remaining part of the peripheral wall once said segment has been removed being formed with a line of weakness to facilitate breaking open of the component.

20. Packaging according to claim 1, comprising an adhesive label adhereable to the exterior of the packaging to cover at least said opener.

21. Packaging according to claim 20, wherein said adhesive label is adhereable to the exterior of the packaging to cover said opener and a significant portion of those parts of the first and second components that abut against one another when assembled.

22. Packaging according to claim 21, wherein operation of said opener causes part of said adhesive label to be removed.

23. Packaging according to claim 1, wherein said assembled components define packaging that is sized to fit through most of the commonly available posting box apertures.

24. Packaging according to claim 23, wherein said packaging has a similar shape to that of a rectangular box, said box being less than 180 mm long, less than 150 mm wide, and less than 50 mm deep.

## Patentansprüche

1. Verpackung (1) für die Verwendung im Transport eines Objekts durch den Postversand, die erste (3) und zweite (5) Komponenten umfasst, die so zusammengefügt werden können, dass ein abgeschlossener Aufbewahrungshohlraum für das zu transportierende Objekt entsteht, wobei die Komponenten (3, 5) jeweils mit entsprechenden Ergänzungsteilen für einen Eingriffmechanismus (21, 23) ausgestattet sind, und die Verpackung des Weiteren einen Öffner umfasst, der bedient werden kann zum Schwächen einer Außenwand einer der Komponenten (5), so dass sich diese Komponente (5) aufbrechen lässt, und die Komponenten (3, 5) sich nicht wieder zusammenbauen lassen, sobald der Öffner (11) bedient worden ist, und wobei die Verpackung (1) durch eine Struktur (45) **gekennzeichnet** ist, die bedient werden kann zum Behindern des Zugangs zu dem Engriffmechanismus (21, 23) von außerhalb der Verpackung.

2. Verpackung nach Anspruch 1, wobei der Engriffmechanismus so ausgeführt ist, dass die Komponenten miteinander verbunden sind, wenn diese zusammengebaut werden und den Hohlraum definieren.

3. Verpackung nach Anspruch 2, wobei der Engriffmechanismus mehrere Stifte umfasst, und jeder Stift einen Haken aufweist, der sich mit einem entsprechenden Schlitz zusammenschließen lässt.

4. Verpackung nach Anspruch 3, die eine Struktur umfasst, die bedient werden kann, um zu verhindern, dass sich die Haken aus den Schlitzen lösen.

5. Verpackung nach Anspruch 4, wobei die Struktur, die verhindert, dass sich die Haken aus den Schlitzen lösen, mehrere Außenwandabschnitte von einer der Komponenten umfasst, in welche die Haken hineinragen, wobei die äußeren Wandabschnitte so angeordnet sind, dass die Schlitze der anderen Komponenten verdeckt werden, wenn die Haken an den Stiften damit zusammengeschlossen werden.

6. Verpackung nach Anspruch 1, wobei eine der Komponenten als frei stehende Basiskomponente konstruiert ist, die ein oder mehrere Stabilisierungselemente umfasst, welche die Basiskomponente in aufrechter Stellung unterstützen können.

7. Verpackung nach Anspruch 1, die einen Behälter innerhalb des Aufbewahrungshohlraumes umfasst, wobei das zu transportierende Objekt in dem Behälter aufbewahrt werden kann.

8. Verpackung nach Anspruch 7, wobei eine Außenwand des Behälters zumindest teilweise lichtdurchlässig oder durchsichtig ist, so dass wenigstens ein Teil des Objektes **dadurch** zu sehen ist.

9. Verpackung nach Anspruch 8, wobei der gesamte Behälter lichtdurchlässig oder durchsichtig ist.

10. Verpackung nach Anspruch 7, wobei der Behälter und eine der Komponenten entsprechende Ergänzungsteile eines beweglichen Engriffmechanismus aufweisen, so dass der Behälter an diese Komponente angefügt werden kann.

11. Verpackung nach Anspruch 10, wobei eine der Komponenten als frei stehende Basiskomponente konstruiert ist, die ein oder mehrere Stabilisierungselemente umfasst, welche die Basiskomponente in aufrechter Stellung unterstützen können, und sich der Behälter an die Basiskomponente anfügen lässt.

12. Verpackung nach Anspruch 11, wobei der Behälter so ausgeführt ist, dass er mit einer Ergänzungsstruktur, die als Teil der zweiten Komponente ausgebildet wird, einen Verschluss bildet, wenn die Komponenten zusammengebaut werden und den Aufbewahrungshohlraum bilden.

13. Verpackung nach Anspruch 1, wobei das zu transportierende Objekt eine Musterflasche umfasst.

14. Verpackung nach Anspruch 13, wobei die Musterflasche in eine absorbierende Abdeckung gehüllt und in einen durchsichtigen Beutel, der sich durch Druck versiegeln lässt, gefasst ist.

15. Verpackung nach Anspruch 14, wobei die absorbierende Abdeckung so ausgeführt ist, dass sie eine Farbänderung anzeigt, wenn der Inhalt der Musterflasche daraus auslaufen sollte und mit der Abdeckung in Kontakt tritt.

16. Verpackung nach Anspruch 15, wobei die Abdeckung mit einem Mittel imprägniert ist, das mit dem ausgelaufenen Inhalt der Musterflasche reagiert und die Farbänderung anzeigt.

17. Verpackung nach Anspruch 1, wobei der Öffner einen Abreißstreifen in einer Außenwand einer der Komponenten umfasst, wobei der Aufreißstreifen bedient werden kann, um mindestens einen Abschnitt der Außenwand zu entfernen.

18. Verpackung nach Anspruch 17, wobei der Abreißstreifen bedient werden kann, um einen allgemein rechteckigen Abschnitt, der sich um den gesamten Umfang der Komponente erstreckt, zu entfernen.

19. Verpackung nach Anspruch 17, wobei der Abreißstreifen bedient werden kann, um einen allgemein rechteckigen Abschnitt, der sich nur um einen Teil des Umfangs der Komponente erstreckt, zu entfernen, und sich, nach Entfernen des Abschnittes, ein Restabschnitt der Außenwand mit einer Schwächelinie bildet, die das Aufbrechen der Komponente erleichtert.

20. Verpackung nach Anspruch 1, die ein Klebeetikett umfasst, das sich an das Äußere der Verpackung kleben lässt, um wenigstens den Öffner abzudecken.

21. Verpackung nach Anspruch 20, wobei sich das Klebeetikett an das Äußere der Verpackung kleben lässt, um den Öffner abzudecken sowie einen wesentlichen Abschnitt jener Teile der ersten und zweiten Komponenten, die aneinander anstoßen, wenn sie zusammengebaut werden.

22. Verpackung nach Anspruch 21, wobei durch die Bedienung des Öffners ein Teil des Klebeetiketts entfernt wird.

23. Verpackung nach Anspruch 1, wobei die zusammengebauten Komponenten eine Verpackung mit einem Format definieren, das durch die meisten allgemein verfügbaren Einwurfschlitze von Postkästen passt.

24. Verpackung nach Anspruch 23, wobei die Verpackung ähnlich einer rechteckigen Schachtel geformt ist, die höchstens 180 mm lang, höchstens 150 mm breit und höchstens 50 mm tief ist.

## Revendications

1. Emballage (1) pour une utilisation pendant le transport d'un article par la poste, l'emballage (1) comportant des premier (3) et second (5) éléments qui peuvent être assemblés pour définir une cavité de rangement enfermée pour l'article à transporter, dans lequel lesdits éléments (3, 5) sont chacun munis de parties complémentaires respectives d'un mécanisme de prise (21, 23), ledit emballage comportant également un dispositif d'ouverture pouvant être actionné pour affaiblir une paroi périphérique d'un dit élément (5), pour permettre audit élément (5) d'être ouvert par rupture, dans lequel lesdits éléments (3, 5) ne peuvent pas être de nouveau assemblés une fois que ledit dispositif d'ouverture (11) a été actionné, l'emballage (1) étant
**caractérisé par** une structure (45) pouvant être actionnée pour entraver l'accès au mécanisme de prise (21, 23) à partir de l'extérieur de l'emballage.

2. Emballage selon la revendication 1, dans lequel ledit mécanisme de prise est tel que lesdits éléments sont bloqués ensemble lorsqu'ils sont assemblés, pour définir ladite cavité.

3. Emballage selon la revendication 2, dans lequel ledit mécanisme de prise comporte une pluralité de languettes portant chacune un cran qui peut venir en prise avec une encoche correspondante.

4. Emballage selon la revendication 3, comportant une structure qui peut être actionnée pour empêcher les crans d'être libérés des encoches.

5. Emballage selon la revendication 4, dans lequel ladite structure pour empêcher les crans d'être libérés des encoches comporte une pluralité de parties de parois périphériques d'un dit élément à l'intérieur duquel lesdits crans s'étendent, lesdites parties de parois périphériques étant agencées pour masquer les encoches sur l'autre desdits éléments lorsque les crans sur les languettes sont en prise avec celles-ci.

6. Emballage selon la revendication 1, dans lequel l'un desdits éléments est configuré comme un élément de base autoporteur, ledit élément de base comportant un ou plusieurs stabilisateurs capables de supporter l'élément de base dans une position verticale.

7. Emballage selon la revendication 1, comportant un récipient agencé à l'intérieur de la cavité de rangement, ledit article à transporter pouvant être rangé à l'intérieur dudit récipient.

8. Emballage selon la revendication 7, dans lequel une paroi périphérique dudit récipient est, au moins en partie, translucide ou transparente de telle sorte qu'au moins une partie dudit article peut être vue à travers celle-ci.

9. Emballage selon la revendication 8, dans lequel la totalité dudit récipient est translucide ou transparente.

10. Emballage selon la revendication 7, dans lequel ledit récipient et l'un desdits éléments incluent des parties complémentaires respectives d'un mécanisme de prise qui peut être actionné pour coupler le récipient à cet élément.

11. Emballage selon la revendication 10, dans lequel ledit un élément est configuré comme un élément de base autoporteur, ledit élément de base comportant un ou plusieurs stabilisateurs capables de supporter l'élément de base dans une position verticale, et ledit récipient est capable d'être couplé audit élément de base.

12. Emballage selon la revendication 11, dans lequel ledit récipient est agencé pour former un joint avec une structure complémentaire formée comme une partie dudit second élément lorsque lesdits éléments sont assemblés pour former ladite cavité de rangement.

13. Emballage selon la revendication 1, dans lequel ledit article à transporter comporte un flacon à échantillons.

14. Emballage selon la revendication 13, dans lequel ledit flacon à échantillons est enveloppé dans un revêtement absorbent et est reçu à l'intérieur d'un sac transparent pouvant être scellé par une presse.

15. Emballage selon la revendication 14, dans lequel ledit revêtement absorbent est agencé pour présenter un changement de couleur dans l'éventualité où le contenu dudit flacon à échantillons présenterait une fuite et viendrait en contact avec ledit revêtement.

16. Emballage selon la revendication 15, dans lequel ledit revêtement est imprégné d'un produit chimique qui réagit avec le contenu ayant fuit dudit flacon à échantillons pour créer ledit changement de couleur.

17. Emballage selon la revendication 1, dans lequel ledit dispositif d'ouverture comporte une bande déchirable dans une paroi périphérique d'un dit élément, ladite bande déchirable pouvant être actionnée pour enlever au moins un segment de la paroi périphérique.

18. Emballage selon la revendication 17, dans lequel ladite bande déchirable peut être actionnée pour enlever un segment généralement rectangulaire s'étendant autour de la totalité de la circonférence de l'élément.

19. Emballage selon la revendication 17, dans lequel ladite bande déchirable peut être actionnée pour enlever un segment généralement rectangulaire s'étendant uniquement autour d'une partie de la circonférence de l'élément, une partie restante de la paroi périphérique, une fois que ledit segment a été enlevé, étant formée en ayant une ligne de faiblesse pour faciliter une ouverture par rupture de l'élément.

20. Emballage selon la revendication 1, comportant une étiquette adhésive pouvant être collée à l'extérieur de l'emballage pour recouvrir au moins ledit dispositif d'ouverture.

21. Emballage selon la revendication 20, dans lequel ladite étiquette adhésive peut être collée à l'extérieur de l'emballage pour recouvrir ledit dispositif d'ouverture et une partie importante de ces parties des premier et second éléments qui viennent en butée l'une contre l'autre lorsqu'elles sont assemblées.

22. Emballage selon la revendication 21, dans lequel un actionnement dudit dispositif d'ouverture amène une partie de ladite étiquette adhésive à être enlevée.

23. Emballage selon la revendication 1, dans lequel lesdits éléments assemblés définissent un emballage qui est dimensionné pour s'adapter à travers la plupart des ouvertures de boîtes postales généralement disponibles.

24. Emballage selon la revendication 23, dans lequel ledit emballage a une forme similaire à celle d'une boîte rectangulaire, ladite boîte ayant moins de 180 mm de longueur, moins de 150 mm de largeur et moins de 50 mm de profondeur.
